# EUROPEAN PATENT APPLICATION

(11) **EP 1 880 741 A1**
(43) Date of publication of application: **23.01.2008**
(21) Application number: 07250628.0
(22) Date of filing: 15.02.2007
(51) Int. Cl.: A61M 5/32, A61M 5/158

(54) **Straight insertion safety infusion set**

(30) Priority: 16.02.2006 US 355642
(71) Applicant: Animas Corporation, West Chester PA 19380 (US)
(72) Inventor: Wojcik, Steven E., Shoreline, WA 98177 (US)
(74) Representative: Tunstall, Christopher Stephen

(57) **Abstract**

An infusion set assembly comprising a cannula housing having a base surface and a septum housing adapted to be pivotally attached to the cannula housing for movement between a position where an axis thereof is substantially perpendicular to the base surface and a position where the axis is substantially parallel to the base surface. The infusion set assembly further comprises an insertion needle assembly including an insertion needle and a cannula supported by the needle assembly with a tip of the insertion needle extending from a distal end of the cannula. An insertion guide housing is configured to support the insertion needle assembly with the needle substantially perpendicular to the base surface and adapted for movement between a position where the needle tip and cannula distal end are spaced from the base surface and a position where the needle tip and cannula distal end extend beyond the base surface.

## Description

### FIELD OF THE INVENTION

The present invention relates generally to an infusion set, and more particularly to a low profile infusion set used for intermittent or continuous delivery of medication, such as insulin to a patient.

### BACKGROUND OF THE INVENTION

Patients who receive intermittent or continuous doses of medication, such as insulin, via subcutaneous injection, often have an infusion set affixed to their skin in a convenient location. Keeping an infusion set fixed in place is discreet, and reduces the need for repeatedly puncturing the skin with a needle, thereby reducing the risk of infection as well as reducing the formation of scar tissue. The infusion set typically includes a housing supporting a tubular cannula with a removable injection needle at one end for penetrating the skin, and a septum at the other end for receiving a needle attached to a supply tube from a medicinal source, e.g., an insulin pump. One well-known conventional infusion set is a "straight set", in which the cannula and injection needle are inserted in an orientation substantially normal to the skin. The straight set requires a relatively short injection needle, which is less intimidating to some patients, and is relatively easy to insert through the skin. But, because the cannula and injection needle are supported to be oriented normal to the skin, the housing must be upright, conspicuous, and relatively bulky, and furthermore, the cannula, rigidly attached to a bottom of the housing can be subject to kinking and occlusion.

Another known infusion set is a low profile angled set, in which the cannula and injection needle are supported in the housing to be oriented at an acute angle with respect to the skin. The housing of the low profile angled set is less bulky and is much more discreet than the housing of the straight set. However, because of the angled insertion, a much longer injection needle is required, and the longer needle is more intimidating and more difficult to insert, and is subject to inadvertent bending.

Additional problems exist with both the conventional straight and angled sets. For example, a relatively long portion of cannula tubing is left exposed. A view of the injection site is often obscured. Adhesive mounting pads used on the sets can be awkward to use, often prematurely contacting the skin, causing wrinkling of the adhesive pad. Moreover, the needle or the cannula often touch non-sterile tissue or clothing prior to insertion, which increases the risk of infection.

Another problem is that in infusion sets in which a self-adhesive pad is used to attach the unit to the skin, the self-adhesive pad must be well supported during insertion to avoid wrinkling the pad. If the user doesn't satisfactorily attach the pad to the skin without wrinkles, the infusion set may need to be removed and replaced. The user is more likely to have a problem applying and smoothing the adhesive pad with the needle/cannula already inserted since they must be careful not to dislodge the cannula. Furthermore, the insertion needle is usually left in place until the adhesive pad is completely attached and may cause pain or discomfort until it is removed. While a relatively small adhesive pad, that is just slightly larger than the infusion set base, would prevent the unsupported edges from drooping and prematurely contacting the skin, a smaller pad provides less adhesion and may allow the infusion set to become detached during use.

### SUMMARY OF THE INVENTION

Accordingly, the present invention is directed to an infusion set that mitigates or substantially obviates one or more of the shortcomings caused by the limitations and disadvantages of the related art.

The features and advantages of the invention will be set forth in the description which follows, and in part will be apparent from the description, or may be learned by practice of the invention. The advantages of the invention will be realized and attained by the apparatus, and the method of practicing the invention, particularly pointed out in the written description and claims below, as well as in the attached drawings.

In accordance with an aspect of the invention, an infusion set assembly is provided with a multiple-part housing. A first portion or cannula housing is removably attachable to a surface of a user's skin. A second portion or septum housing is pivotally attached to the cannula housing, pivotable between a first position above the cannula housing and substantially normal to the surface of the skin and a second position alongside the cannula housing portion and substantially parallel to the skin surface. An elongated tubular cannula is provided, having a first end and a second end. An injection needle is removably mounted in the cannula and extends from the first end. The infusion set assembly is configured such that the injection needle and the first end of the cannula penetrate the skin surface at an injection site in an orientation that is substantially normal to the skin surface. After insertion of the cannula, the insertion needle is removed and the septum housing is pivoted to the second position.

In accordance with another aspect of the invention, the cannula is initially supported by the insertion needle spaced from a bottom surface of the cannula housing. An adhesive assembly is provided on the bottom surface of the cannula housing for adhering the cannula housing portion to the skin. With the cannula initially maintained spaced from the adhesive surface, the user can fully attach the infusion set base and smooth the adhesive to the skin before the needle/cannula is inserted. If the adhesive pad is not satisfactorily attached or not smoothed without wrinkles, the infusion set can be removed without cannula/needle insertion.

In accordance with yet another aspect of the invention, a disposable insertion guide housing portion is provided for supporting the cannula housing portion and the insertion handle portion above the injection site prior to injection, allowing the user to preposition the infusion set generally perpendicular to and above the skin surface at the injection site. The infusion set can be pre-packaged with the disposable insertion guide housing portion, ready to use, right off the shelf without needing to be assembled by the user. The guide housing portion also maintains the insertion needle hidden from view during the entire insertion process to lessen the user's anxiety, particularly in the case of children.

In accordance with a further aspect of the invention, an insertion needle or solid trocar initially passes through the interior of the soft cannula such that its sharp cutting edges extend beyond the distal end of the cannula. The proximal end of the needle is attached to an insertion needle handle which is mounted within an insertion guide housing which positions and holds the needle and cannula distal end above the cannula housing. With the needle and cannula distal end spaced from the attachment surface, the cannula housing is attached to the skin at the insertion site on the skin. Thereafter, the insertion needle handle is pressed towards the skin such that the needle and cannula distal end are guided towards the cannula housing by the insertion guide housing. The needle and cannula distal end pass through the opening in the cannula housing, penetrate the skin, and are inserted perpendicularly into the tissue. The insertion needle is then withdrawn into the insertion guide housing leaving only the distal end of the cannula in the subcutaneous tissue. The insertion guide housing can then be removed and discarded with the insertion needle safely shielded inside. During the entire insertion process the insertion needle is never exposed.

In accordance with a further aspect of the invention, the proximal end of the septum housing is pivoted approximately 90 degrees and latched to the main body of the cannula housing so as to prevent further rotation or movement. As the septum housing is pivoted, the portion of the cannula between the distal end of the septum housing and the opening in the base of the cannula housing is bent in a smooth arc over a mandrel on the cannula housing. The mandrel controls the bend radius of the cannula in the latched position to further prevent the cannula from kinking. Clearance between the cannula and opening in the main body allows the cannula to flex if the inserted cannula and the housing bottom are not perpendicular without kinking the cannula. Once the septum housing is latched, a needle hub assembly may be attached to the cannula housing assembly.

In accordance with still another aspect of the invention, the septum housing supports the cannula and is initially detached from the cannula housing. Upon depression of the insertion needle handle, pivot pins on the septum housing engage mating pivot holes in the cannula housing. The pivot pins snap into the holes, pivotally interconnecting the septum housing to the cannula housing.

In accordance with yet another aspect of the invention, the septum housing remains pivotally-attached to the cannula housing. The septum and cannula are mounted coaxially within a separate cannula cartridge which is mountable on the insertion needle with the needle extending from the cannula distal end. The cannula cartridge is initially mounted above and axially-aligned with a stepped bore in the septum housing. The axis of the stepped bore in the septum housing is aligned with the opening in the cannula housing and is perpendicular to the flat base of the cannula housing. When the insertion handle is pressed towards the skin, the cannula cartridge is advanced such that the cartridge enters the stepped bore of the septum housing, and the needle and cannula distal end pass through the opening in the bottom of the cannula housing penetrating the surface of the skin. As the handle is depressed, the cannula cartridge engages and is retained within the septum housing. After the insertion handle is retracted and removed, the septum housing can then be pivoted parallel to the surface of the skin and latched.

It is to be understood that both the above general description and the following detailed description are exemplary and explanatory, and are intended to explain the principles of the claimed invention. The accompanying drawings are included to provide a further understanding of the invention and are incorporated and constitute part of the specification, illustrating presently preferred embodiments of the invention.

### BRIEF DESCRIPTION OF THE DRAWINGS

Fig. 1 is a perspective view of an infusion set assembly in accordance with a first exemplary embodiment of the invention;
Fig. 2 is an exploded perspective view of the infusion set assembly of Fig. 1;
Fig. 3 is a cross-sectional view along line 3-3 of Fig. 1;
Fig. 4 is a cross-sectional view similar to Fig. 3 with the insertion handle depressed and the cannula fully inserted;
Fig. 5 is a cross-sectional view similar to Fig. 4 with the insertion handle retracted after the cannula has been fully inserted;
Fig. 6 is a cross-sectional view similar to Fig. 5 with the insertion handle retracted into the insertion guide housing which is removed from the cannula housing after the cannula has been fully inserted;
Fig. 7 is a perspective view of the cannula housing as illustrated in Fig. 6;
Fig. 8 is a perspective view of the cannula housing with the cannula fully inserted and the septum housing in the folded and latched position;
Fig. 9 is a cross-sectional view along the line 9-9 of Fig. 8;
Fig. 10 is a top view of a needle hub assembly about to be removably attached to the inserted infusion set of Fig. 8;
Fig. 11 is an exploded view of an alternative embodiment of the insertion set assembly of the present invention;
Fig. 12 is a cross-sectional side view of the insertion set assembly of Fig. 11;
Fig. 13 is a cross-sectional view similar to Fig. 12 with the insertion handle depressed, the cannula cartridge assembly inserted into the septum housing, and the cannula fully inserted into the subcutaneous tissue;
Fig. 14 is a cross-sectional view similar Fig. 13 with the insertion handle retracted, the cannula cartridge assembly inserted into the septum housing, and the cannula fully inserted into the subcutaneous tissue;
Fig. 15 is a cross-sectional view similar to Fig. 14 with the insertion handle retracted and the insertion guide housing removed from the cannula housing assembly;
Fig. 16 is a perspective view of an alternate insertion guide housing in accordance with an exemplary embodiment of the invention with the insertion needle handle fully retracted; and
Fig. 17 is a perspective view of the insertion guide housing of Fig.16 with the cover closed.

### DETAILED DESCRIPTION OF THE INVENTION

Preferred features of embodiments of this invention will now be described with reference to the figures. It will be appreciated that the spirit and scope of the invention is not limited to the embodiments selected for illustration. Also, it should be noted that the drawings are not rendered to any particular scale or proportion. It is contemplated that any of the configurations and materials described hereafter can be modified within the scope of this invention.

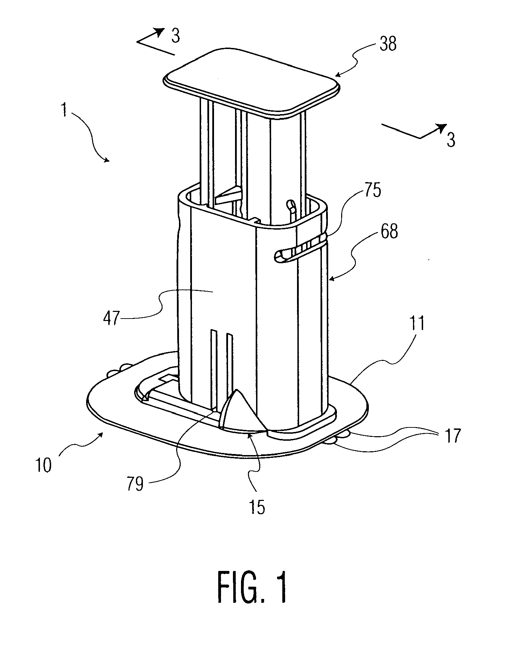

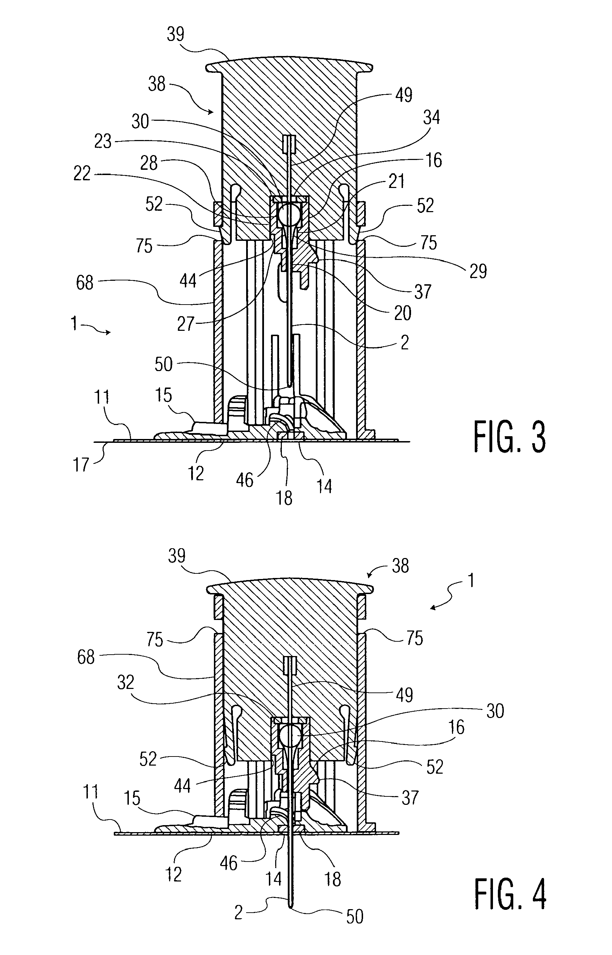

Referring to Figs. 1-9, infusion set assembly 1 that is a first exemplary embodiment of the present invention is shown. Infusion set assembly 1 generally includes cannula housing assembly 10, septum housing assembly 80, insertion guide housing 68, and insertion needle assembly 38. Desirably, infusion set assembly 1 is supplied in a sterile package in the pre-assembled configuration as shown in Figs. 1 and 3.

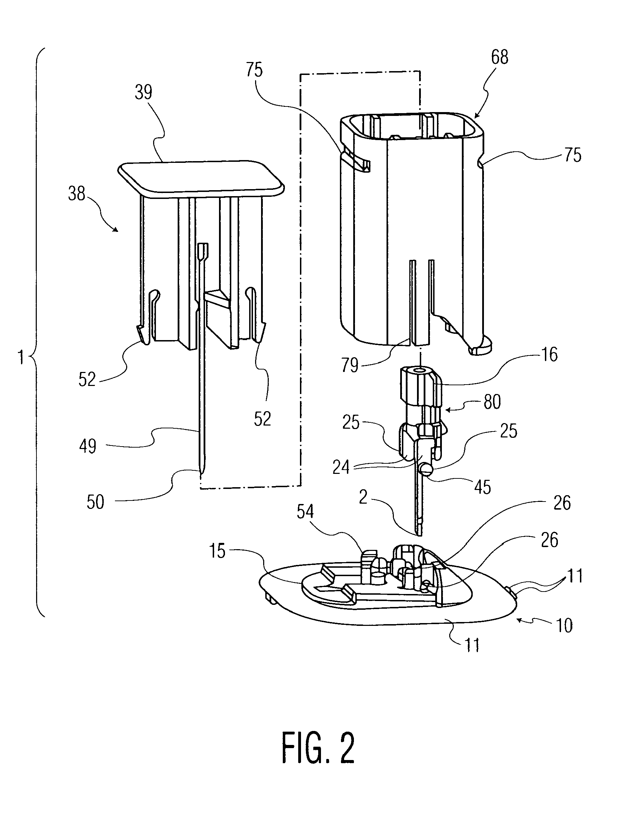

Referring to Fig.2-3, septum housing assembly 80 includes septum housing 16 which is configured to support cannula 2. Septum housing 16 is desirably manufactured from molded plastic, but may be any suitable material. A series of stepped bores 20, 21, 22 and 23 are defined in septum housing 16 and extend axially from a distal end of septum housing 16 to a proximal end thereof. Cannula 2 extends through stepped bore 21 and extends from the distal end of septum housing 16. Ferrule 29 desirably includes a tapered distal portion 27 and a cylindrical proximal portion 28. Ferrule 29 extends from stepped bore 22 to stepped bore 21 wherein tapered distal portion 27 extends within a flared proximal portion of cannula 2. Ferrule distal portion 27 compresses cannula 2 against bore 20 of septum housing 16, thereby creating a fluid-tight seal.

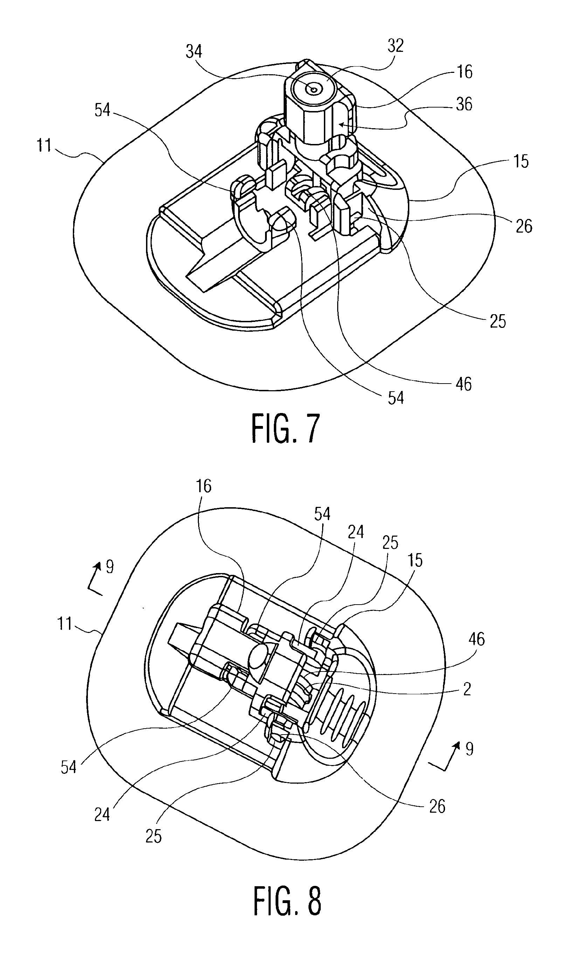

Septum 30 is compressed slightly to fit within the inner bore of ferrule proximal portion 28 and thereby seals the proximal end of cannula 2. Septum 30 may be spherical, as illustrated, or any other configuration to seal the ferrule proximal portion 28. Septum retainer 32 is positioned in septum housing bore 23 to retain septum 30 and ferrule 29 within septum housing 16. Septum retainer 32 may be press-fit, adhesive-bonded, ultrasonically welded or otherwise retained in bore 23. Septum retainer 32 has a concentric septum aperture 34, see Fig. 7, configured to facilitate passage of an insertion needle or infusion needle as will be described hereinafter. The details of septum 30, ferrule 29 and cannula 2 are for illustrative purposes only and many other methods commonly known in the art may be used for securing and providing a septum seal over the proximal end of cannula 2.

Septum housing assembly 80 is configured to be pivotally connected to cannula housing assembly 10 after insertion of cannula 2 into a user. Cannula housing 15 of cannula housing assembly 10 includes a pair of pivot holes 26 configured to snap-fittingly receive pivot pins 25 on septum housing 16 to retain septum housing assembly 80 pivotally connected to cannula housing assembly 10. Pivot holes 26 are desirably through holes opening both inwardly and outwardly, with the pivot pins 25 being received through the inward portions of holes 26. Septum housing pivot pins 25 are located at the ends of flexible arms 24 on opposed sides of cannula housing 16. Flexible arms 24 are configured to flex inward to permit each pivot pin 25 to snap into mating engagement with a respective pivot hole 26 in cannula housing 15, as shown in Fig. 2. The forward edge of each pivot pin 25 is desirably beveled, as indicated at 45, to further facilitate positioning of pivot pins 25 into the respective holes 26 more easily. In the present embodiment, pivotal interconnection between septum housing assembly 80 and cannula housing assembly 10 occurs after insertion of cannula 2, as will be described in more detail hereinafter.

Cannula housing 15 further includes a substantially planar bottom surface 12 with opening 14 extending therethrough for passage of cannula 2. Elastomeric disk 18 or the like is desirably mounted about opening 14 and includes a through bore for passage of cannula 2. Elastomeric disk 18 is configured to seal around the outside diameter of cannula 2 after insertion. Opening 14 may be sized to receive elastomeric disk 18 as shown, or alternatively, elastomeric disk 18 may be positioned against bottom surface 12 of cannula housing 15 and retained thereagainst, for example, by mounting pad 11 which is described hereinafter. Alternatively, elastomeric disk 18 may be omitted and the diameter of opening 14 through cannula housing 15 may be made just slightly larger than the outer diameter of cannula 2.

To protect cannula 2, cannula housing 15 includes a cannula guide or curved mandrel 46, positioned to support a portion of cannula 2 proximate opening 14. Cannula guide 46 provides an arcuate path for cannula 2. As septum housing 16 is pivoted about pivot pins 25 to a second position, as described hereinafter, cannula guide 46 supports an intermediate portion of cannula 2 along a gradual curve. The arcuate path is approximately a fraction of a 90° bend, with a radius in the range of approximately 1-4 mm and desirably about 2.25 mm. This slight curve prevents kinking of cannula 2.

Self-adhesive mounting pad 11 is attached to bottom surface 12 of cannula housing 15. The adhesive surface of mounting pad 11 is initially covered by a removable backing paper or liner 17. Liner 17 may be divided into multiple parts to make removal easier. Since cannula 2 is initially maintained spaced from bottom surface 12, as shown in Fig. 3, the user can fully attach cannula housing assembly 10 and smooth mounting pad 11 to the skin before cannula 2 is inserted. If mounting pad 11 is not satisfactorily attached or not smoothed without wrinkles, cannula housing assembly 10 can be removed, and possibly reapplied, without disruption to an inserted cannula.

Referring to Figs. 1-4, insertion needle assembly 38 is configured to insert cannula 2 into the user's skin. Insertion needle assembly 38 includes handle 39 with insertion needle or trocar 49 attached thereto. Handle 39 is configured for guided movement within insertion guide housing 68. As shown in Fig. 3, in axial cross-section, handle 39 has a generally square or rectangular shape with a rounded top surface. However, the cross-section may have various configurations configured to complement insertion guide housing 68. While it is desirable that handle 39 and insertion guide housing 68 are configured to prevent handle 39 from rotating within insertion guide housing 68, such is not necessary provided septum housing assembly 80 is prevented from rotating relative to cannula housing assembly 10 or a universal connection, for example, a ball and socket connection, is provided between septum housing assembly 80 and cannula housing assembly 10. While handle 39 may have a solid outer cross-section, the illustrated example is defined by a series of ribs which reduces the overall mass and improves the molding characteristics of handle 39. Flexible barbs 52 on opposed sides of handle 39 are configured to engage notches 75 in insertion guide housing 68. As shown in Fig. 3, engagement of barbs 52 in notches 75 holds insertion needle assembly 38 in a retracted position until handle 39 is depressed. Barbs 52 are configured to flex inward as handle 39 is depressed, as shown in Fig. 4.

Insertion needle 49 is adhesive-bonded or otherwise connected to handle 39 and is configured to be passed coaxially through retainer 32, septum 30, ferrule 29, and cannula 2 such that needle tip 50 extends, for example, 2 or 3 mm, beyond the distal end of the cannula 2. Insertion needle 49 may be a standard beveled needle as is common in the art or a solid trocar. In addition to being easier to manufacture, sharp tip 50 of a trocar can be shorter than a comparable needle since the bevels extend in 3 or 4 facets from the needle centerline to the outer edge rather than across the entire diameter of the needle. A shorter insertion needle tip 50 length will not protrude as far beyond the end of the cannula 2 and may reduce the risk of contacting underlying muscle tissue during insertion.

Insertion needle 49, cannula 2 and septum 30 are desirably configured such that friction between insertion needle 49 and septum 30 and cannula 2 is sufficient to retain septum housing assembly 80 to insertion needle assembly 38 without any additional retaining mechanism, however, an additional retaining mechanism may be provided if desired. Notch 44 in insertion needle handle 39 orients septum housing assembly 80 and aligns pivot pins 25 with pivot holes 26 in cannula housing 15.

Insertion guide housing 68 rests on the top surface of cannula housing 15 and is initially attached to cannula housing assembly 10 by flexible barbs 79 which engage the outward portions of pivot holes 26 in cannula housing 15. Insertion guide housing can be released from cannula housing 15 by flexing barbs 79 outward. Insertion guide housing 68 desirably has flat sides 47 configured to be gripped to prevent rotation of infusion set assembly 1 during insertion, thus offering more control over the subsequent orientation of the infusion set on the surface of the skin. Insertion guide housing 68 may be molded in one piece from plastic such as polycarbonate, polypropylene, or other plastic or may be manufactured utilizing deferent techniques and different materials.

Having described the components of infusion set assembly 1, its operation will now be described with respect to Figs. 1 and 3-9. Infusion set assembly 1 is supplied in sterilized packaging in the configuration shown in Figs. 1 and 3 with insertion guide housing 68 attached to cannula housing assembly 10, septum housing assembly 80 mounted to insertion needle assembly 38, and handle 39 in the retracted position. Cannula 2 and insertion needle 49 are hidden from view and fully protected within insertion guide housing 68.

Referring to Fig. 3, barbs 52 on handle 39 engage notches 75 in insertion guide housing 68 to hold cannula 2 and sharp tip 50 of insertion needle 49 above opening 14 in cannula housing 15. The user then removes backing paper 17 to expose the adhesive surface of mounting pad 11. The user may carefully position, adhere, and smooth mounting pad 11 on the skin without the discomfort or anxiety associated with an inserted needle.

Referring to Fig. 4, the top of insertion needle handle 39 is pressed, thereby disengaging barbs 52 from notches 75 and advancing needle 49 and septum housing assembly 80 towards the skin. Insertion needle 49 and cannula 2 pass through opening 14 in cannula housing 15 into the subcutaneous tissue. Pivot pins 25 on septum housing 16 flex inward upon contact with the top of cannula housing 15 until pins 25 engage the inward portions of pivot holes 26. Pivot pins 25 desirably make an audible "click" as pins 25 snap outward into pivot holes 26 to alert the user that cannula 2 and needle 49 are fully inserted and septum housing 16 is securely connected to cannula housing 15.

Infusion set assembly 1 provides a ready to use, preloaded infusion set. Since the force to insert needle 49 is well below 0.5 pound, needle 49 and cannula 2 may easily be inserted with only finger pressure. Since the speed of insertion is controlled by the user, needle insertion may be stopped at any time if the user feels discomfort such as when penetrating muscle tissue. However, unlike insertion by hand, insertion guide housing 68 allows cannula housing 15 to be attached to the skin before inserting cannula 2, thus ensuring that cannula 2 is inserted perpendicular to the surface of the skin.

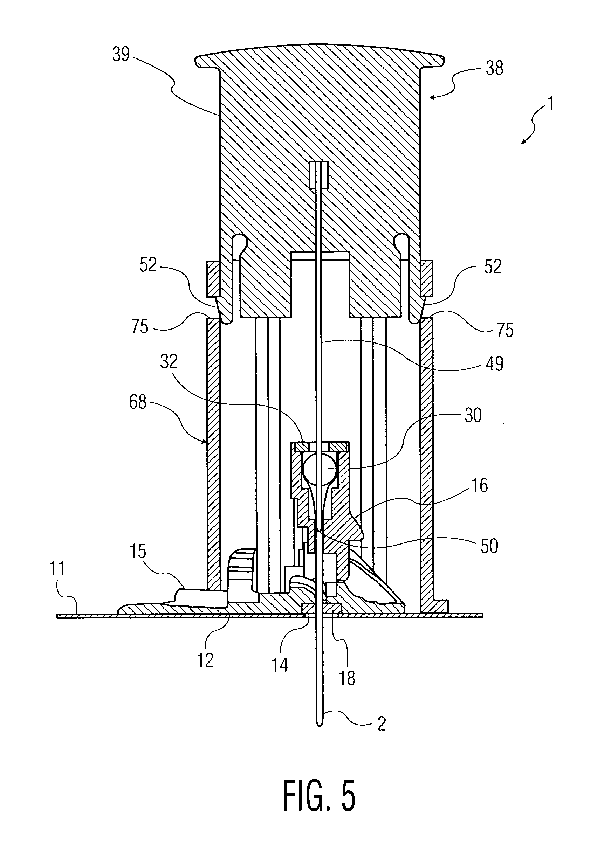

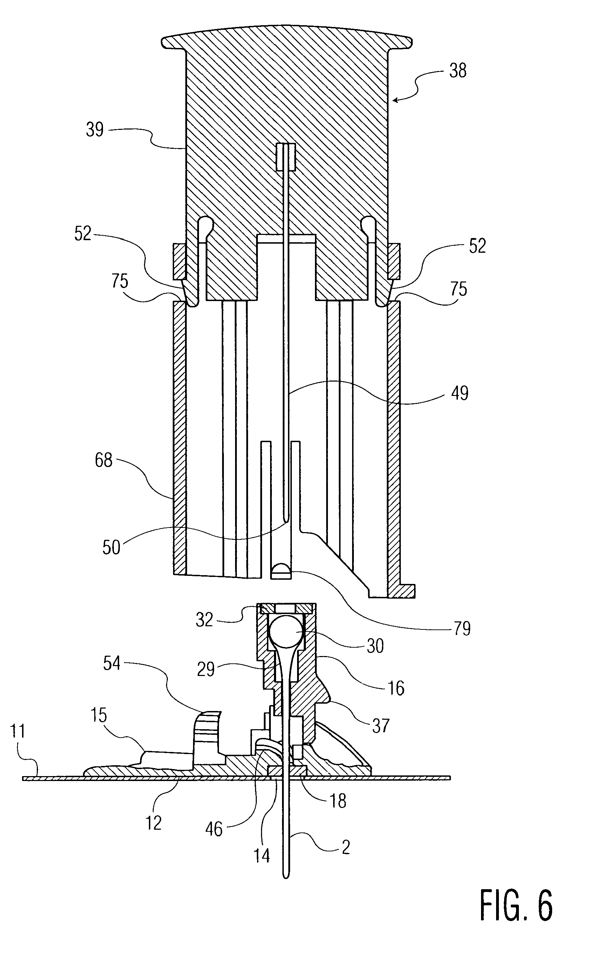

As pivot pins 25 engage pivot holes 26, pivot pins 25 extend through holes 26 such that barbs 79 on insertion guide housing 68 are pushed outward by pivot pins 25, thereby unlatching insertion guide housing 68 from cannula housing 15. Insertion needle handle 39 may be retracted away from the skin into insertion guide housing 68 until barbs 52 engage notches 75 as shown in Fig. 5. Alternatively, insertion guide housing 68 can be removed from cannula housing 15 with handle 39 depressed, and thereafter, needle 49 retracted. In either case, insertion guide housing 68 is removed and discarded with insertion needle 49 safely shielded inside insertion guide housing 68 as shown in Fig. 6.

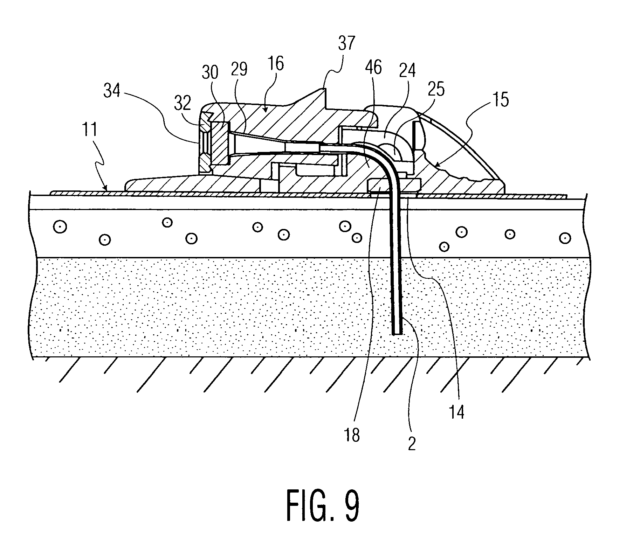

Fig. 7 shows cannula housing assembly 10 after insertion guide housing 68 and needle 49 have been removed. At this time, septum housing assembly 80 is still generally perpendicular to cannula housing bottom surface 12. Septum housing 16 is then pivoted from this upright position until it latches in the flattened position as shown in Figs. 8 and 9. Latching barbs 54 on cannula housing 15 engage mating notches on the sides of the septum housing 16. Desirably, septum housing 16 snaps into position with an audible "click". As septum housing 16 pivots from the upright insertion position to the flattened, latch position, the portion of exposed cannula 2 between opening 14 in cannula housing 15 and bore 20 of septum housing 16 is bent in a smooth radius over curved mandrel 46. Mandrel 46 desirably has a groove which is wider than the outer diameter of cannula 2 to accommodate cannula misalignment. Since septum housing pivot pins 25 are located at the ends of flexible arms 24, opening 14 in cannula housing 15 is visible once septum housing 16 is in the flattened, latched position, allowing the infusion site to be viewed. Finger depression 42 is provided on the top of main body 15 to allow the user to hold the cannula housing assembly 10 in place while septum housing 16 is folded and latched.

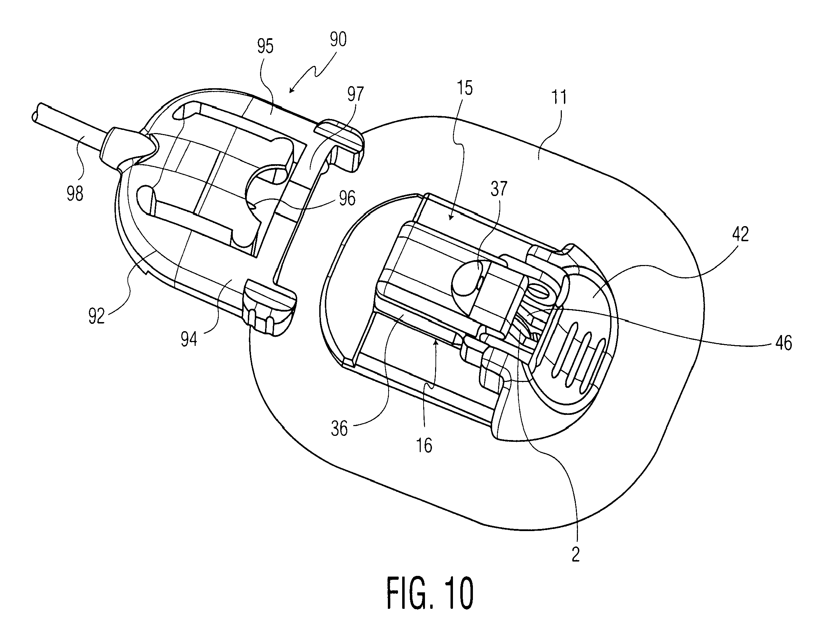

Once cannula 2 is inserted and septum housing 16 latched in the folded position, infusion set assembly 1 is ready for connection to an infusion source, for example, an infusion pump, through a needle hub assembly. A first exemplary embodiment of a needle hub assembly 90 useable with infusion set assembly 1 is illustrated in Fig. 10. Needle hub assembly 90 is configured to be removably attached to septum housing 16.

Needle hub assembly 90 includes a substantially flat housing 92, a resilient band 97, flexible arms 94 and 95, and a needle 96. The needle hub assembly 90 includes guide rails (not shown) extending along a lower surface of housing 92, inward of flexible arms 94 and 95. The guide rails are configured to align with and slide into grooves 36 in the sides of septum housing 16. Sliding of the guide rails into grooves 36 guides needle 96 through septum retainer 32 and septum 30 such that needle 96 terminates in ferrule 29.

To retain needle hub assembly 90 in engagement with septum housing 16, resilient band 97 extends between flexible arms 94 and 95 and is configured to flex over and be retained by a retaining bump 37 on septum housing 16. Retaining bump 37 desirably has a ramped profile when viewed from the side (see Fig. 3) such that resilient band 97 slides freely over retaining bump 37 as needle housing assembly 90 is connected to septum housing portion 16. Resilient band 97 may make an audible click to give the user positive acknowledgment that needle hub assembly 90 and septum housing 16 are locked together. To remove needle hub assembly 90, arms 94 and 95 are squeezed together such that resilient band 97 flexes upward to a central height greater than the height of retaining bump 37. Needle hub assembly 90 is slid off of septum housing portion 16, thereby removing needle 96 from septum 30. Other types of engaging and locking assemblies may also be used. For example, a set of complementary barbs and notches (not shown) can be provided on opposing surfaces of needle hub assembly 90 and septum housing 16. Other assemblies may also be utilized.

Flexible tube 98 is attached to needle 96 and projects from a rear end of needle hub assembly 90. Tube 98 can extend directly to a medication source, such as an insulin pump, or to an appropriate fitting such as a Luer fitting, which can be connected to an external infusion pump (not shown) or another medicine source. Operation of the medication source supplies medicine through tube 98, through needle 96 and through cannula 2 to deliver the of medicine to the user.

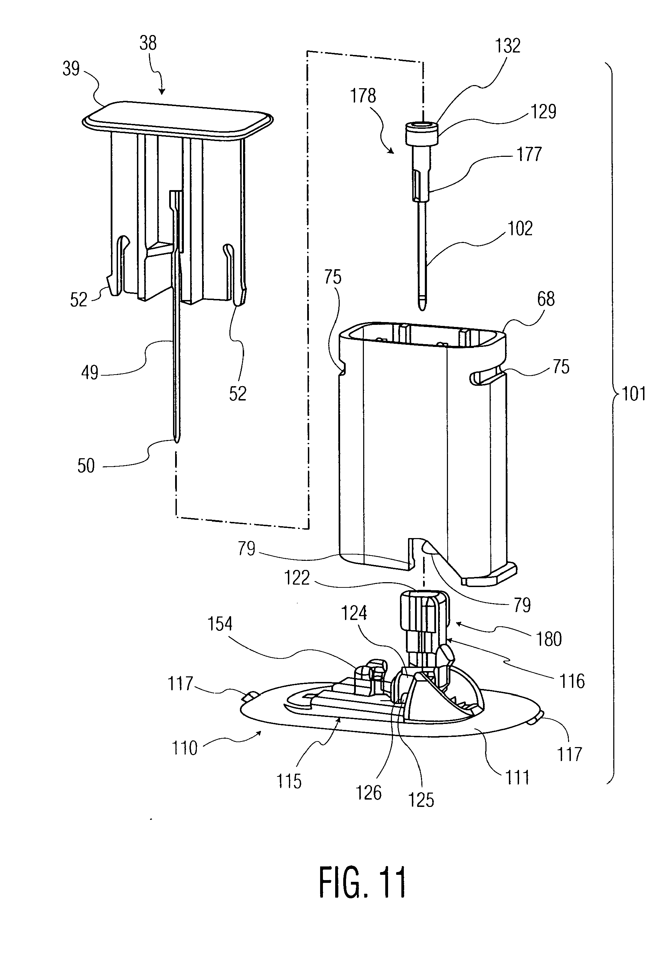

Referring to Figs. 11-15, infusion set assembly 101 that is a second exemplary embodiment of the present invention is illustrated. Infusion set assembly 101 is similar to the previous embodiment, however, septum housing assembly 180 is initially pivotally attached to cannula housing assembly 110 while cannula 102, ferrule 129 and septum 130 are mounted in a separate insertable cannula cartridge 178.

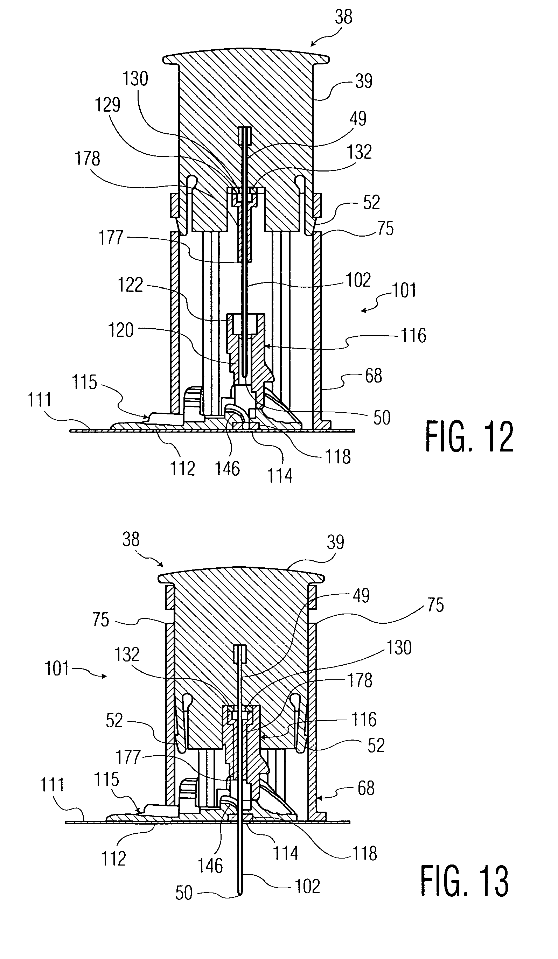

As shown in Figs. 11-15, insertion needle handle assembly 38 and insertion guide housing 68 in the present embodiment are essentially the same as in the first exemplary embodiment. Insertion needle assembly 38 includes handle 39 with insertion needle or trocar 49 attached thereto. Handle 39 is configured for guided movement within insertion guide housing 68. Flexible barbs 52 on opposed sides of handle 39 are configured to engage notches 75 in insertion guide housing 68. As shown in Fig. 12, engagement of barbs 52 in notches 75 holds insertion needle assembly 38 in a retracted position until handle 39 is depressed. Barbs 52 are configured to flex inward as handle 39 is depressed, as shown in Fig. 13. Insertion needle 49 is adhesive-bonded or otherwise connected to handle 39 and is configured to be passed coaxially through insertable cannula cartridge 178, as will be described.

Cannula housing assembly 110 is also similar to cannula housing assembly 10 of the previous embodiment and includes cannula housing 115 with a substantially planar bottom surface 112 with opening 114 extending therethrough for passage of cannula 102. Elastomeric disk 118 or the like is desirably mounted about opening 114 and includes a through bore for passage of cannula 102. Elastomeric disk 118 is configured to seal around the outside diameter of cannula 102 after insertion.

Self-adhesive mounting pad 111 is attached to bottom surface 112. The adhesive surface of mounting pad 111 is initially covered by a removable backing paper or liner 117. Liner 117 may be divided into multiple parts to make removal easier. As in the previous embodiment, cannula 102 will initially be maintained spaced from bottom surface 112, as shown in Fig. 12, and the user can fully attach cannula housing assembly 110 and smooth mounting pad 111 to the skin before cannula 102 is inserted. If mounting pad 111 is not satisfactorily attached or not smoothed without wrinkles, cannula housing assembly 110 can be removed, and possibly reapplied, without disruption to an inserted cannula.

As in the previous embodiment, cannula housing 115 includes pivot holes 126 configured to receive pivot pins 125 extending from flexible arms 124 of septum housing 116. In the current embodiment, in the initial configuration as shown in Fig. 12, septum housing 116 is pivotally connected to cannula housing 115 via pivot pins 125. Septum housing 116 is initially oriented such that the axis thereof is substantially perpendicular to bottom surface 112 of the cannula housing 115.

Stepped bores 120 and 122 extend through septum housing 116 and are configured to receive insertable cannula cartridge 178. Referring to Figs. 11 and 12, cartridge 178 is configured to receive cannula 102, ferrule 129, septum 130 and septum retainer 132. Cartridge 178 is desirably a molded plastic assembly, but may be manufactured using various materials and various methods. Cannula 102 extends from the distal end of cartridge 178. Ferrule 129 is attached to and supports cannula 102. Ferrule 129 may be a separate component positioned within cartridge 178, or alternatively, may be formed as a portion of cartridge 178 as illustrated. Septum 130 is positioned in ferrule 129 to seal the proximal end of cannula cartridge 178. Septum retainer 132 is bonded, ultrasonically welded or otherwise secured in cannula cartridge 178 to retain septum 130 and cannula 102. Cannula cartridge 178 desirably has one or more outwardly extending barbs 177 on the distal end thereof to retain the cannula cartridge 178 in septum housing 116 by snap fit, however, various methods of securing cartridge 178 in place can be used.

The assembled cannula cartridge 178 is mounted on insertion needle assembly 38 with insertion needle 49 passing coaxially through retainer 132, septum 130, ferrule 129, and cannula 102 such that needle tip 50 extends beyond the distal end of cannula 102, for example, by 2 or 3 mm. Needle 49, septum 130 and cannula 102 are desirably configured such that friction between insertion needle 49 and septum 130 and cannula 102 is sufficient to hold cannula cartridge 178 on insertion needle assembly 38 without any additional locking mechanism, however, a locking mechanism may be provided if desired. Cannula cartridge 178 is generally symmetrical about the cannula axis so it is not necessary to key or otherwise orient cannula cartridge 178 on insertion needle assembly 38.

Insertion guide housing 68 is attached to cannula housing 115 in a manner similar to that of the previous embodiment. Since pivot pins 125 on septum housing 116 are already engaged in pivot holes 126, pivot holes 126 are made deeper than in the previous embodiment to allow barbs 79 on insertion guide housing 68 and pivot pins 125 on septum housing 116 to simultaneously be positioned in pivot holes 126. To remove insertion guide housing 68, sides 81 of housing 68 are squeezed together, springing barbs 79 outward. Insertion guide 68 may then be lifted off cannula housing 115.

Having described the components of infusion set assembly 101, operation thereof will be described with reference to Figs. 12-15. Infusion set assembly 101 is desirably supplied in a sterilized package in the configuration as shown in Fig. 12. Cannula cartridge 178 is positioned on insertion needle 49 with needle tip 50 extending from the distal end of cannula 102. Septum housing 116 is pivotally connected to cannula housing 115 and positioned with the axis of septum housing 116 substantially perpendicular to cannula housing bottom surface 112. Handle 39 is in a retracted position with a portion of needle 49 extending into septum housing bores 120, 122, but not extending beyond bottom surface 112. To utilize infusion set assembly 101, backing paper 117 is removed from the adhesive surface of mounting pad 111. The user may carefully position, adhere, and smooth mounting pad 111 on the skin without the discomfort or anxiety associated with an inserted needle.

Pressing the insertion needle handle 39 towards the skin disengages barbs 52 from notches 75 of insertion guide housing 68, thereby advancing insertion needle 49 and cannula cartridge assembly 178 towards septum housing 116. Sharp tip 50 of needle 49 passes through opening 114 in cannula housing 115, penetrating the skin. As barbs 177 on cannula cartridge 178 enter bore 120 in septum housing 116, barbs 177 flex inward towards cannula 102.

Cannula cartridge 178 is configured such that at approximately the moment insertion needle 49 and cannula 102 are fully inserted, barbs 177 have passed completely through bore 120 and spring outward and latch cannula cartridge 178 in septum housing 116 as shown in Fig. 13. Desirably, springing of barbs 177 makes an audible "click" to alert the user that cannula 102 has been fully inserted and cannula cartridge 178 is secured in septum housing 116.

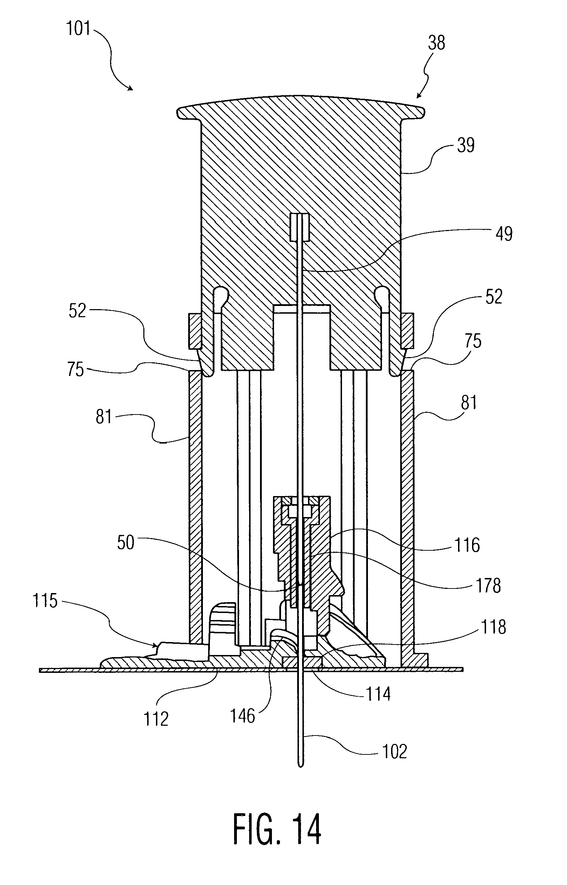

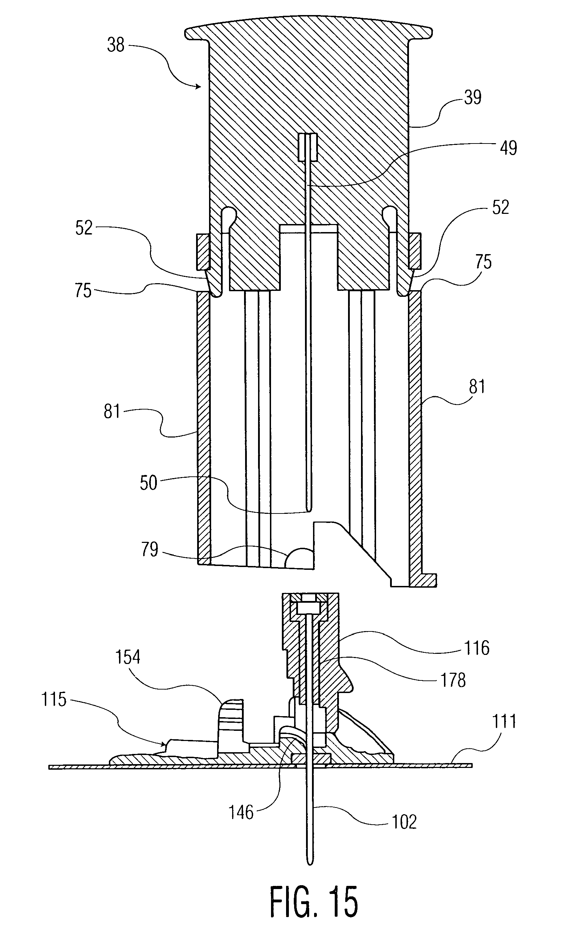

Insertion needle handle 39 is then retracted until barbs 52 again engage notches 75 in insertion guide housing 68, as shown in Fig. 14. Squeezing opposed sides 81 of insertion guide housing 68 releases barbs 79 from pivot holes 126 of cannula housing 115 such that insertion guide housing 68 can be removed. Insertion guide housing 68 can then be discarded with needle tip 50 safely shielded inside guide housing 68 as shown in Fig. 15. Once guide housing 68 is removed, infusion set assembly 101 is in the same configuration as illustrated in Fig. 7 with respect to the first exemplary embodiment. Septum housing 116 may then folded and latched via barbs 154 in a manner similar to that shown in Figs. 8 and 9 and as described in the previous embodiment. As in the previous embodiments, as septum housing 116 pivots from the upright insertion position to the flattened, latch position, a portion of cannula 102 is bent in a smooth radius over curved mandrel 146. Needle hub assembly 90 may then be attached to septum housing 116 in a manner similar to that described with respect to Fig. 10, to permit the flow of medication.

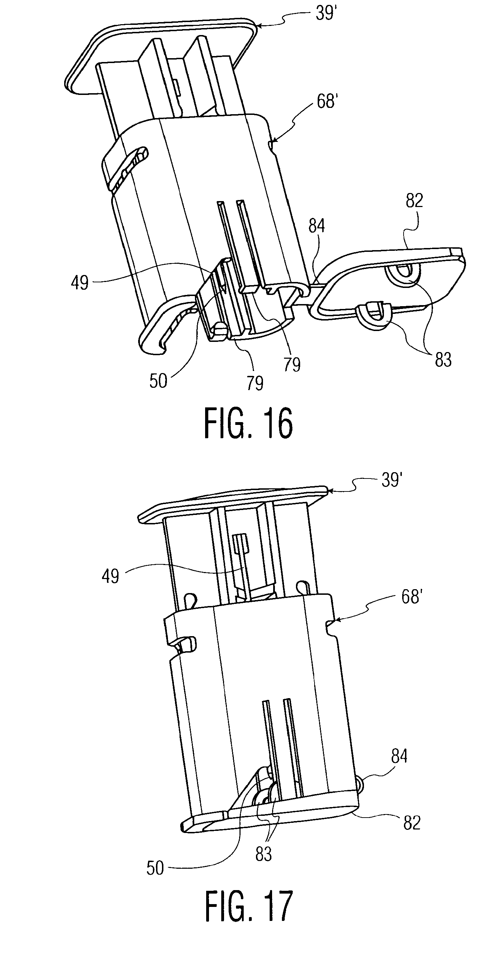

An alternate insertion guide housing 68' with a cover 82 is illustrated in Figs. 16-17. In the present embodiment, the length of the insertion guide housing 68' and handle 39' are shorter than in the previous embodiments in order to minimize the overall size of the device as well as the bulk of disposable material. The use of this version is similar to the previously described embodiments, however, by shortening these components, the sharp tip 50 of needle 49 is closer to the open end of insertion guide housing 68' in the retracted position. To protect the user from an accidental needle stick, cover 82 can be snapped to the bottom of insertion guide housing 68'. Cover 82 can be a separate component, or desirably, integrally attached to insertion guide housing 68', for example, by integral hinge 84 as shown. Cover 82 includes attachment loops 83 configured to engage insertion guide housing barbs 79 to latch cover 82 in the closed position. Other latching mechanisms may also be utilized.

While the described invention has been taught with specific reference to the above-described embodiments, those skilled in the art will recognize that changes can be made in form and detail without departing from the spirit and the scope of the invention.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. An infusion set assembly comprising:
a cannula housing having a base surface adapted to be removeably attached to a user's skin;
an insertion needle assembly including an insertion needle;
a cannula supported by the needle assembly with a tip of the insertion needle extending from a distal end of the cannula;
an insertion guide housing configured to support the insertion needle assembly relative to the cannula housing with the needle substantially perpendicular to the base surface and adapted for movement between a first needle assembly position where the needle tip and cannula distal end are spaced from the base surface and a second needle assembly position where the needle tip and cannula distal end extend beyond the base surface; and.
a septum housing configured to receive and support the cannula relative to the cannula housing.

2. The infusion set assembly of claim 1 wherein the septum housing has an axial axis and the septum housing is adapted to be pivotally attached to the cannula housing and moveable between a first septum housing position where the axial axis is substantially perpendicular to the base surface and a second septum housing position where the axis is substantially parallel to the base surface.

3. The infusion set assembly of claim 2 wherein the septum housing is pivotally attached to the cannula housing when the needle assembly is in the first needle assembly position.

4. The infusion set assembly of claim 3 further comprising a cannula cartridge configured to support the cannula on the needle assembly and further configured to be received and retained in the septum housing axial bore upon movement of the needle assembly to the second needle assembly position.

5. The infusion set assembly of claim 4 wherein the cannula cartridge includes at least one radially outwardly extending barb configured to snap-fittingly engage the septum housing upon movement of the needle assembly to the second needle assembly position.

6. The infusion set assembly of claim 4 wherein a septum is sealingly received in a proximal end of the cartridge assembly to seal the proximal portion of the cannula.

7. The infusion set assembly of claim 6 wherein a friction force between the needle, the septum and the cannula retains the cannula on the needle assembly when the needle assembly is in the first needle assembly position.

8. The infusion set assembly of claim 6 wherein the cannula cartridge defines or further comprises a ferrule between the septum and the cannula proximal portion.

9. The infusion set assembly of claim 2 wherein the cannula proximal portion is positioned in the septum housing bore and the septum housing is disconnected and spaced from the cannula housing when the needle assembly is in the first needle assembly position.

10. The infusion set assembly of claim 9 wherein the septum housing and cannula are supported on the needle assembly when the needle assembly is in the first needle assembly position.

11. The infusion set assembly of claim 10 wherein a septum is sealingly received in a proximal end of the septum housing to seal the proximal portion of the cannula.

12. The infusion set assembly of claim 11 wherein a friction force between the needle, the septum and the cannula retains the cannula and septum housing on the needle assembly when the needle assembly is in the first needle assembly position.

13. The infusion set assembly of claim 12 wherein a ferrule extends in the septum housing bore between the septum and the cannula proximal portion.

14. The infusion set assembly of claim 10 wherein the cannula housing includes a pair of pivot holes and the septum housing includes a pair of pivot pins configured to be received in the pivot holes as the needle assembly is moved to the second needle assembly position.

15. The infusion set assembly of claim 2 wherein the cannula housing includes a cannula guide configured to support a portion of the cannula along an arcuate path when the septum housing is in the second septum housing position.

16. The infusion set assembly of claim 15 wherein the arcuate path comprises at least a fraction of a 90° turn.

17. The infusion set assembly of claim 2 further comprising a latching mechanism for fixing the septum housing to the cannula housing when the septum housing portion is in the second septum housing position.

18. The infusion set assembly of claim 1 further comprising an adhesive assembly attached to the base surface.

19. The infusion set assembly of claim 18 wherein the adhesive assembly comprises an adhesive pad and a removable backing.

20. The infusion set assembly according to claim 1 further comprising a needle hub assembly removably attachable to the septum housing and connectable to a source of medication.

21. The infusion set assembly of claim 20 wherein the needle hub assembly includes an infusion needle positionable within the cannula.

22. The infusion set assembly of claim 1 wherein the insertion needle assembly includes at least one flexible barb configured to engage a corresponding notch in the insertion guide housing to maintain the needle assembly in the first needle assembly position.

23. The infusion set assembly of claim 1 wherein the insertion guide housing includes at least one flexible barb configured to engage a hole in the cannula housing to retain the insertion guide housing connected to the cannula housing.

24. The infusion set assembly of claim 23 wherein the insertion guide housing and the insertion needle assembly are removable from the cannula housing and septum housing after insertion of the cannula.

25. The infusion set assembly of claim 24 wherein the needle is configured to be retained within the insertion guide housing after removal from the cannula housing and septum housing.

26. The infusion set assembly of claim 25 wherein a cover is configured to be positioned over a distal end of the insertion guide housing to contain the needle within the insertion guide housing.

27. An infusion set assembly comprising:
a cannula housing having a base surface;
an insertion needle assembly including an insertion needle;
a cannula supported by the needle assembly with a tip of the insertion needle extending from a distal end of the cannula;
an insertion guide housing configured to support the insertion needle assembly with the needle substantially perpendicular to the base surface and adapted for movement between a first needle assembly position wherein the needle tip and cannula distal end are spaced from the base surface and a second needle assembly position wherein the needle tip and cannula distal end extend beyond the base surface; and
a septum housing having an axial bore configured to receive and support a proximal portion of the cannula, the septum housing being pivotally attached to the cannula housing for movement between a first septum housing position where an axis of the bore is substantially perpendicular to the base surface and a second septum housing position where the axis is substantially parallel to the base surface.

28. The infusion set assembly of claim 27 further comprising a cannula cartridge configured to support the cannula on the needle assembly and further configured to be received and retained in the septum housing axial bore upon movement of the needle assembly to the second needle assembly position.

29. The infusion set assembly of claim 28 wherein the cannula cartridge includes at least one radially outwardly extending barb configured to snap-fittingly engage the septum housing upon movement of the needle assembly to the second needle assembly position.

30. An infusion set assembly comprising:
a cannula housing having a base surface;
a septum housing having an axial bore, the septum housing being adapted to be pivotally attached to the cannula housing and moveable between a first septum housing position where an axis of the bore is substantially perpendicular to the base surface and a second septum housing position where the axis is substantially parallel to the base surface;
a cannula having a distal end and a proximal portion, the proximal portion supported in the septum housing axial bore;
an insertion needle assembly including an insertion needle, the insertion assembly configured to support the cannula and septum housing with a tip of the insertion needle extending from the cannula distal end; and
an insertion guide housing configured to support the insertion needle assembly with the needle substantially perpendicular to the base surface and adapted for movement between a first needle assembly position where the needle tip and cannula distal end are spaced from the base surface and a second needle assembly position where the needle tip and cannula distal end extend beyond the base surface.

31. The infusion set assembly of claim 30 wherein the cannula housing includes a pair of pivot holes and the septum housing includes a pair of pivot pins configured to be received in the pivot holes as the needle assembly is moved to the second needle assembly position.
